# FASCICULE DE BREVET EUROPEEN

(11) **EP 0 810 984 B1**
(45) Date de publication et mention de la délivrance du brevet: **20.09.2000**
(21) Numéro de dépôt: 96904136.7
(22) Date de dépôt: 14.02.1996
(51) Int. Cl.: C07C 15/08

(54) **PROCEDE DE SEPARATION DU P-XYLENE COMPORTANT UN PRETRAITEMENT PAR HYDROGENATION SELECTIVE ET PAR DE LA TERRE ACTIVEE**
VERFAHREN ZUR TRENNUNG VON P-XYLOL, DAS EINE VORBEHANDLUNG DURCH SELEKTIVE HYDRIERUNG UND EINE BEHANDLUNG MIT AKTIVER ERDE UMFASST
METHOD FOR SEPARATING PARA-XYLENE BY PREPROCESSING USING SELECTIVE HYDROGENATION AND ACTIVATED EARTH

(30) Priorité: 21.02.1995 FR 9502082
(43) Date de publication de la demande: 10.12.1997
(73) Titulaire: INSTITUT FRANCAIS DU PETROLE, 92506 Rueil-Malmaison Cédex (FR)
(72) Inventeur: JOLY, Jean-François, F-69006 Lyon (FR); CAMERON, Charles, F-75005 Paris (FR); RENARD, Pierre, F-78860 Saint-Nom-La-Breteche (FR); MONTECOT, Françoise, F-78340 Les Clayes-sous-Bois (FR); COSYNS, Jean, F-78580 Maule (FR); LEGER, Gérard, F-69300 Caluire (FR)
(86) Numéro de dépôt international: FR9600238
(87) Numéro de publication internationale: WO9626170

(56) Documents cités:
- US-A- 4 118 429
- US-A- 4 224 141
- US-A- 5 284 992

## Description

La présente invention concerne un procédé de séparation et de récupération du paraxylène à partir d'une charge à traiter contenant un mélange de xylènes.

Elle concerne aussi un nouveau procédé de traitement des coupes d'hydrocarbures riches en composés aromatiques, et plus particulièrement un procédé d'élimination des dioléfines et de réduction et éventuellement d'élimination des oléfines de mélanges d'hydrocarbures riches en composés aromatiques par réaction d'hydrogénation au moyen de catalyseurs particuliers.

Elle concerne plus particulièrement la production de para-xylène très pur, notamment pour la synthèse d'acide téréphtalique utilisé principalement dans l'industrie textile.

Les mélanges d'hydrocarbures riches en composés aromatiques sont utilisés dans les complexes pétrochimiques, où après divers traitements de séparation et de purification ils conduisent à l'obtention de benzène, de toluène, d'éthylbenzène, de méta-xylène, d'ortho-xylène, de para-xylène, d'éthylbenzène et de styrène.

Les coupes riches en composés aromatiques contenant des teneurs variables en dioléfines et en oléfines proviennent, en général : des procédés de distillation des hydrocarbures bruts tels que le pétrole brut, le condensat de gaz naturel, la houille ; des procédés thermiques tels que le vapocraquage de naphta ; des procédés dédiés à la production des aromatiques à partir des coupes aliphatiques légères (C₃-C₅ et plus particulièrement C₃ et C₃/C₄), aliphatiques C₆ et C₆/C₇, et les coupes de naphta lourd (>C₆ pour des divers procédés de reformage catalytiques) ; et des procédés de transformation de produits aromatiques tels que les procédés de trans-alkylation et d'isomérisation d'ortho et meta-xylènes en para-xylène.

Le procédé de reformage catalytique est le procédé majeur qui permet de produire lesdits mélanges riches en composés aromatiques. À l'origine, le reformage catalytique était pratiqué dans deux types d'installations, selon son utilisation en raffinage ou en pétrochimie. Par la suite, cette distinction liée à la sévérité des conditions opératoires s'est estompée.

Maintenant, pour répondre aux contraintes énergétiques accrues, les industriels recherchent à nouveau des procédés plus spécifiques. Ils ont ainsi recours en raffinage à des unités de reformage catalytique opérant à haute sévérité, mais avec une plus grande stabilité de fonctionnement et des rendements améliorés en essences, et en pétrochimie à l'optimisation de la production en aromatiques (benzène, toluène et xylènes) par l'emploi de réacteur fonctionnant à basse pression.

L'utilisation de reformeurs opérant à haute sévérité s'est accompagnée d'une augmentation de la teneur en oléfines et dioléfines dans les réformats. En effet le nombre de brome du réformat stabilisé peut atteindre une valeur maximum dépassant 7000 mg de brome par 100 g de produit pour les unités opérant à très faible pression. La présence de ces oléfines et dioléfines est particulièrement préjudiciable aux procédés de séparation des aromatiques. Par exemple, les oléfines et dioléfines ont tendance à polymériser dans les solvants utilisés pour les extractions. Des traitements de purification utilisant des silico aluminates naturels, généralement activés, (par exemple : Attapulgite, Bentonites et Montmorillonites activés par traitement en présence d'acides) sont couramment utilisés. Ces matériaux de purification sont généralement appelés des terres activées.

L'utilisation des traitements de purification à la terre activée présente de nombreux inconvénients parmi lesquels on peut citer : une faible durée de vie (4 à 6 mois en général et parfois aussi faible que 1 mois pour des charges ayant des concentrations importantes en oléfines, > 0,6 % poids), une activité catalytique faible (vitesses spatiales horaires comprises entre 0,5 à 3 volumes de charge/volume de catalyseur/heure), la très grande difficulté pour purifier des charges contenant plus de 1,5 % en poids d'oléfines et de dioléfines, ce qui limite la sévérité de fonctionnement du reformeur. La purification de la charge s'accompagne de la production de composés de haut poids moléculaire par réaction d'alkylation des aromatiques. Ces produits sont obligatoirement séparés par la suite. Par ailleurs, l'utilisation de ces terres activées pose des problèmes vis-à-vis de la protection de l'environnement. En effet, elles ne sont pas économiquement régénérables et sont donc éliminées dans des décharges avec leurs produits aromatiques résiduels toxiques.

Les schémas conventionnels de production de composés aromatiques comprenant de 6 à 8 atomes de carbone par molécule sont complexes. Ils comprennent une succession d'étapes de séparation, de purification et de procédés catalytiques. Un complexe aromatique de production de para-xylène et d'ortho-xylène peut être décrit comme comprenant au moins l'enchaînement des étapes décrites ci-après.

La source d'aromatiques, c'est-à-dire le reformat stabilisé est introduit dans une colonne de séparation qui permet d'obtenir en tête une coupe C₇⁻ qui contient du benzène et du toluène, cette colonne est couramment appelée "déheptaniseur". En fond du déheptaniseur est soutiré une coupe aromatique C₈⁺ qui est purifiée par passage sur au moins un lit de terre activée. Cette purification a pour objectif d'éliminer la majeure partie des oléfines et dioléfines de cette coupe C₈⁺. La durée de vie de ces lits de terre activée étant relativement faible, deux lits ou plus de deux lits sont disposés en parallèle de façon à pouvoir passer de l'un à l'autre pour opérer la décharge de la terre usée sans arrêt de la production de coupe C₈⁺ purifiée.

À la sortie du traitement à la terre, la coupe C₈⁺ aromatique est introduite dans une colonne de séparation dans laquelle on extrait une coupe C₈ aromatique en tête (xylène + éthylbenzène) et une fraction lourde contenant l'ortho-xylène et les composés de plus haut poids moléculaire que l'ortho-xylène en fond. Il est d'usage d'opérer une seconde séparation sur cette dernière coupe pour récupérer de l'orthoxylène en tête de cette seconde colonne et une coupe aromatique C₉⁺ en fond dont on pourra éventuellement séparer les aromatiques en C₉ pour produire, par réaction de transalkylation avec du benzène, une quantité supplémentaire de para-xylène et d'ortho-xylène.

La coupe aromatique C₈ obtenue en tête de la première colonne, qui contient les trois isomères xylènes (ortho-xylène, méta-xylène et para-xylène) et l'éthylbenzène est envoyée vers une unité de séparation des xylènes, par exemple utilisant des tamis moléculaires ou un procédé de cristallisation, de laquelle on obtient d'une part du para-xylène et d'autre part une coupe C₈ aromatique. Cette dernière est introduite dans une unité catalytique d'isomérisation, éventuellement après addition d'hydrogène, dans laquelle les xylènes et éventuellement l'éthylbenzène sont isomérisés pour produire un mélange de C₈ aromatique proche de l'équilibre thermodynamique, et qui contient donc du para-xylène. Un schéma d'isomérisation est décrit dans le brevet US 4224141.

À la sortie du réacteur d'isomérisation, on utilise généralement une colonne de séparation des fractions légères C₅⁻ produites dans le réacteur d'isomérisation puis une autre colonne de séparation qui permet de recueillir en fond une coupe C₈⁺ qui contient les C₈ aromatiques proches de l'équilibre thermodynamique et les composés aromatiques lourds issus des réactions de transalkylation du procédé d'isomérisation. Cette coupe C₈⁺ aromatique peut contenir des oléfines et dioléfines. Il est donc d'usage de traiter cette coupe par au moins un lit de terre activée avant de la recycler vers le déheptaniseur qui traite le reformat. Un tel schéma est décrit dans la demande de brevet FR-94/15896 qui combine en outre un procédé d'enrichissement en para-xylène par adsorption sélective ou cristallisation à basse température, une purification par cristallisation à au moins un étage à haute température et un procédé d'isomérisation. On est donc amené à disposer d'au moins deux réacteurs et les rejets de terre usagée deviennent de plus en plus problématiques pour des raisons de pollution de l'environnement.

Le brevet US-4 118 429 décrit un procédé d'hydrogénation d'oléfines dans les effluents d'unités d'isomérisation des composés aromatiques utilisant comme catalyseur un métal choisi parmi les métaux : ruthénium, rhodium, palladium, osmium, iridium, platine ou un mélange de ces métaux. L'objectif indiqué de cette invention est d'améliorer la récupération de para-xylène au traitement par l'adsorbant. Il est indiqué dans l'exemple qu'un effluent exempt d'oléfines est obtenu après traitement à 177°C sous 9,7 bar de pression, à une vitesse spatiale de 3 h⁻¹ et sur un catalyseur composé de 0,375 % poids de platine sur alumine. La perte en composés aromatiques n'est jamais précise dans la description de l'invention ; mais cette perte est significative. En effet, l'hydrogénation des coupes riches en xylènes par des métaux du groupe VIII conduit à une perte significative de xylènes sous forme de diméthylcyclohexanes comme indiqué ci-après.

Par ailleurs l'effluent contient encore des monooléfines qui perturbent le traitement en aval, notamment une adsorption sur tamis moléculaire.

L'objet de l'invention est de remédier aux inconvénients mentionnés ci-dessus.

Plus précisément, l'invention concerne un procédé de séparation et de récupération du p-xylène à partir d'une charge à traiter contenant un mélange de xylènes, dans lequel on fait circuler une partie au moins d'une charge contenant un mélange de xylènes dans une zone dite d'enrichissement (14) adaptée à enrichir une première fraction en p-xylène et délivrant une deuxième fraction appauvrie en p-xylène, on fait circuler ladite deuxième fraction dans une zone d'isomérisation (19) et on récupère un isomérat (20) que l'on recycle vers la zone d'enrichissement, le procédé étant caractérisé en ce que l'on fait circuler au moins une partie choisie dans le groupe formé par la charge initiale, l'isomérat et leur mélange, dans au moins une zone d'hydrogénation sélective (3) en présence d'hydrogène, puis dans au moins une zone (8) de traitement à la terre, on réalise une étape de séparation (5) de l'hydrogène en excès dans au moins une zone de séparation, avant ou après ledit traitement à la terre, et on récupère un effluent que l'on envoie dans la zone d'enrichissement (14).

Plusieurs variantes peuvent être proposées. Selon une première variante, l'isomérat et la charge à traiter peuvent être hydrogénés dans la zone (3) d'hydrogénation, puis soumis au traitement à la terre (8), avant ou après séparation (5) de l'hydrogène puis l'effluent résultant est envoyé dans la zone d'enrichissement (14).

Selon une deuxième variante, la charge à traiter peut être hydrogénée dans la zone (3) d'hydrogénation, puis soumise au traitement (8) à la terre, avant ou après séparation (5) de l'hydrogène, l'isomérat (20) étant recyclé entre les zones d'hydrogénation et de traitement à la terre, et on récupère l'effluent qui est envoyé dans la zone d'enrichissement (14).

Selon une troisième variante, la charge à traiter peut être hydrogénée dans la zone (3) d'hydrogénation, puis soumise à un traitement à la terre avant ou après séparation de l'hydrogène et l'isomérat (20) peut être recyclé après la zone de traitement (8) à la terre de la charge hydrogénée, le-dit isomérat ayant préalablement subi un traitement à la terre dans une zone de traitement (24) différente.

Selon une quatrième variante, l'isomérat peut être hydrogéné dans la zone (3) d'hydrogénation, puis soumis au traitement (8) à la terre avant ou après séparation de l'hydrogène, et la charge à traiter, d'où on a éliminé au moins en partie des dioléfines, peut être introduite en amont de la zone d'enrichissement (14).

Le procédé selon la présente invention permet de purifier des mélanges d'hydrocarbures riches en composés aromatiques et ne présente pas les inconvénients inhérents aux traitements conventionnels utilisant comme système de purification uniquement des traitements aux terres activées. Le procédé selon la présente invention peut purifier de nombreuses charges hydrocarbures dont la teneur et la nature des aromatiques peut varier dans une plage très large. De plus, ledit procédé n'est pas limité par la teneur en oléfines et dioléfines des charges à purifier. Parmi les avantages dudit procédé on peut citer :
- la possibilité de traiter des charges caractérisées par des teneurs en oléfines et dioléfines élevées, par exemple de 0,1 à plus de 3 % poids d'oléfines et avantageusement moins de 0,3 % poids de diènes,
- la teneur en composés aromatiques dans les effluents hydrogénés reste presque inchangée,
- l'absence de production de composés de haut poids moléculaire par l'étape d'hydrogénation, les oléfines éventuellement transformées et les dioléfines sont converties en paraffines,
- la nuisance vis-à-vis de l'environnement est significativement réduite car le catalyseur est régénérable et les oléfines non hydrogénées sont les moins gênantes pour les procédés en aval du traitement. La durée de vie de la terre activée est nettement prolongée, les quantités de terres mises à la décharge sont donc très inférieures par rapport aux procédés conventionnels.

Par exemple, on a constaté que lorsqu'on traitait la charge selon le procédé de l'invention et lorsqu'on recyclait l'isomérat en amont de la zone de traitement à la terre on pouvait n'utiliser qu'un seul réacteur de traitement à la terre, voire le contourner lors des opérations de changement de terre.

Selon une caractéristique de l'invention, on peut simultanément à la séparation de l'hydrogène en excès résultant de l'hydrogénation sélective, réaliser une séparation des composés C₇⁻.

On peut selon une autre variante, affiner la séparation en effectuant la séparation de l'hydrogène dans une première zone de séparation, puis la séparation des composés C₇⁻ dans au moins une autre zone de séparation.

Il est encore possible d'améliorer la séparation de ces composés selon l'utilisation envisagée des coupes intermédiaires. Ainsi, on peut effectuer la séparation de l'hydrogène dans une première zone de séparation, puis la séparation des composés C₅⁻ dans une deuxième zone de séparation, et la séparation des composés C₇⁻ restants dans une troisième zone de séparation.

On peut également réaliser la séparation de l'hydrogène dans une première zone de séparation, puis la séparation des composés C₄⁻ dans une deuxième zone de séparation, et la séparation des composés C₇⁻ restants dans une troisième zone de séparation.

L'hydrogène séparé peut au moins en parti être recyclé dans la zone d'hydrogénation sélective.

Ces trois dernières alternatives permettent avantageusement de récupérer des fractions intermédiaires, coupe heptane, coupe pentane, coupe butane sensiblement pures et notamment débarrassées des dioléfines ou de produits plus lourds qui peuvent colmater les tamis moléculaires des réacteurs.

Des composés légers, notamment des hydrocarbures C₇⁻ étant formés lors de l'étape d'isomérisation, on peut effectuer leur séparation, par distillation par exemple, après l'étape d'isomérisation.

Généralement, la charge à traiter contenant des teneurs variables en dioléfines et en oléfines est un réformat brut, un réformat brut d'où ont été éliminés au moins en partie les composés C₃⁻, un réformat brut d'où ont été éliminés au moins en partie les composés C₄⁻, un réformat brut d'où ont été éliminés au moins en partie les composés C₅⁻, une coupe C₈ riche en composés aromatiques contenant une majeure partie de xylènes et leurs mélanges.

Selon une autre caractéristique de l'invention, on peut faire circuler l'effluent à enrichir dans au moins une colonne de distillation dite "rerun" située à l'entrée de la zone d'enrichissement en para-xylène, pour recueillir un mélange xylènes-éthylbenzène et un mélange ortho-xylène-produits lourds.

Selon une première variante, cette zone d'enrichissement peut être au moins une zone de cristallisation à très basse température qui délivre, après une étape de séparation, un liquide (ladite deuxième fraction) que l'on isomérise dans la zone d'isomérisation et des cristaux que l'on fond qui sont une partie au moins de ladite première fraction.

Selon une deuxième variante particulièrement avantageuse, la zone d'enrichissement en para-xylène peut être une zone d'adsorption sélective contenant un adsorbant zéolithique, on réalise une adsorption sélective de la charge, en présence d'un solvant de désorption, on récupère ladite première fraction enrichie en para-xylène et la seconde fraction appauvrie en para-xylène, on isomérise ladite seconde fraction dans une zone d'isomérisation contenant un catalyseur d'isomérisation dans des conditions adéquates pour produire un isomérat contenant du para-xylène et on recycle au moins en partie l'isomérat vers la zone d'adsorption.

Selon une autre caractéristique de l'invention, l'hydrogénation sélective des dioléfines peut être effectuée en présence d'un catalyseur contenant au moins un élement du groupe VIII dans lequel du soufre a été introduit préalablement au passage de la charge à hydrogéner. Ce soufre peut être introduit par traitement du catalyseur au moyen d'un composé soufré dans la zone d'hydrogénation ou bien, il peut être introduit dans le catalyseur au moyen d'un agent soufré, préalablement au chargement du catalyseur dans la zone d'hydrogénation.

Plus précisément, dans ce dernier cas, au moins un agent soufré à base d'un composé organique renfermant du soufre peut être incorporé dans le catalyseur préalablement à son emploi et préalablement à son activation par de l'hydrogène.

L'agent soufré peut être choisi parmi au moins un thioéther R₁-S-R₁, au moins un thiol R₁-SH, au moins un disulfure organique R₁-S-S-R₂ ou HO-R₁-S-S-R₂-OH et au moins un polysulfure organique R₁-(S)n-R₂, les radicaux organiques R₁ et R₂ étant identiques ou différents, pris séparément ou en combinaison et n est un nombre entier de 1 à 20.

Avantageusement, cet agent peut être choisi dans le groupe de l'hydrogène sulfuré, le diméthylsulfure, le diméthyldisulfure ou le ditertiononylpolysulfure (TPS-37, Elf Atochem).

ces agents soufrés et leur incorporation dans le catalyseur sont décrits dans le brevet FR-2 664 610 et dans la demande de brevet FR-a-2 729 968.

Les catalyseurs préférés contiennent au moins un métal du groupe du nickel, du platine et du palladium et plus particulièrement de 0,1 à 1 % poids de platine ou de palladium par rapport au support ou de 5 à 70 % poids de nickel par rapport au support.

Il est généralement incorporé de 0,05 à 10 % de soufre exprimé en poids par rapport à la masse de catalyseur et plus particulièrement de 0,05 à 1 % de soufre lorsque le palladium est utilisé, notamment lorsque la teneur en dioléfines à extraire de la charge est faible.

Les conditions de l'hydrogénation sélective sont, en règle générale, les suivantes :
- Température : 20 à 250°C, de préférence 80 à 180°C,
- Pression : 4 - 50 bar, de préférence 10 à 40 bar,
- VVH : 0,2 à 25 h⁻¹, de préférence 2 à 15 h⁻¹,
- Rapport molaire : hydrogène/monooléfines + polyoléfines et/ou acétyléniques : 0,3 - 100, de préférence 1 - 50.

Dans ces conditions, la conversion des composés aromatiques de la charge est inférieure à 0,15 % et de préférence inférieure à 0,1 %. Les charges qui sont adaptées pour la présente invention, présentent un nombre de brome de 10.000 à 100 mg par 100 g de produit, par exemple.

Le procédé comporte habituellement au moins un réacteur catalytique d'hydrogénation dans lequel la charge à traiter est majoritairement à l'état liquide à l'entrée du réacteur. De façon préférée, au moins 80 % poids de la charge est à l'état liquide à l'entrée du réacteur.

L'hydrogène et la charge à traiter sont injectés en courant ascendant ou descendant dans le réacteur, qui est de préférence à lit fixe de catalyseur.

Après hydrogénation, la conversion des dioléfines est en général supérieure à 75 % et le plus souvent supérieure à 90 %. En revanche, la conversion des monooléfines est sensiblement moins importante (15 à 40 % par exemple).

Un traitement à la terre activée est donc généralement réalisé selon les conditions décrites dans les demandes de brevet FR-A-2 728 893 et FR- A-2 728 894 de la Demanderesse. Par exemple, les conditions d'adsorption ou d'élimination des composés indésirables sont les suivantes :
- Température : 100 à 300°C, de préférence 160 à 230°C,
- Vitesse spatiale horaire : 1 à 8, de préférence 1 à 4 (volume horaire de charge par volume de terre),
- Type de terre : aluminosilicates naturels activés, par exemple la terre référencée F54 - chez Engelhard,
- Pression 3 à 100 bar, de préférence 4 à 20 bar.

La fraction de xylènes débarrassée de la majeure partie des impuretés peut être enrichie par adsorption sélective sur tamis moléculaire, comme décrit dans les brevets de la Demanderesse US-A 5 284 992 ou par cristallisation à basse température comme décrit dans les brevets US-A 5 329 061 et US-A 2 866 833. La fraction enrichie peut ensuite être purifiée dans une zone dite de purification par au moins une cristallisation, à haute température, et de préférence dans une zone de purification comprenant au moins deux étages de cristallisation à haute température, comme décrit dans les demandes de brevet: FR-A-2 728 893, FR-A-2 728 894 et FR-A- 2 729 660.

Une partie au moins de la liqueur mère résultant de l'étage le plus froid de la zone de purification peut être alors recyclée à l'entrée de la zone d'hydrogénation sélective où elle peut être mélangée avec la charge initiale et/ou l'isomérat. Selon une autre variante, elle peut être recyclée en n'importe quel point en amont de la zone d'enrichissement, par exemple avant ou après le réacteur de traitement à la terre.

L'invention sera mieux comprise au vu des figures ci-dessous 1 à 6 illustrant de manière schématique et non limitative plusieurs variantes du procédé. Les organes du dispositif ayant des fonctions bien définies ont les mêmes références dans toutes les figures.

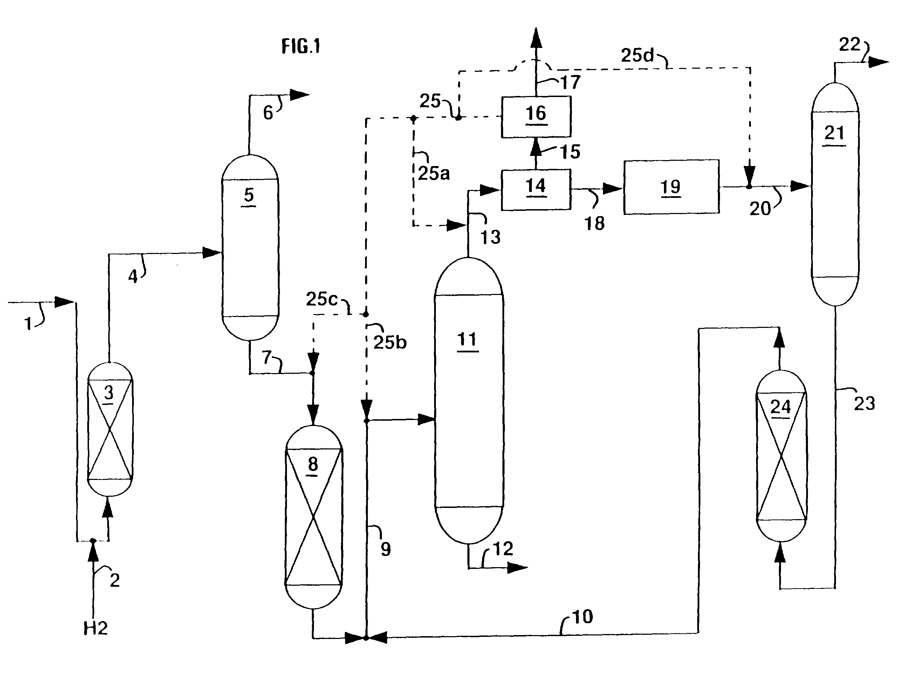

Selon la figure 1, la charge aromatique, un réformat brut par exemple, débarrassée des hydrocarbures C₃⁻ et C₄⁻ (non représenté sur la figure) est introduite par une ligne 1, en présence d'hydrogène éventuellement recyclé via une ligne 2 dans un réacteur 3 d'hydrogénation sélective comprenant un lit fixe de catalyseur (alumine de transition) contenant 0,1 à 1 % poids par rapport au support de palladium préalablement soufré ex-situ et contenant 0,05 à 1 % de soufre (poids par rapport à la masse de catalyseur). L'effluent hydrogéné ne contenant sensiblement plus de dioléfines est envoyé par une ligne 4 dans un séparateur 5 (déheptaniseur principal) d'où sont extraits par une ligne 6, de l'hydrogène en excès et des hydrocarbures C₇⁻ tel que le benzène et le toluène dont la pureté est améliorée. Du fond du séparateur 5, une ligne 7 contenant un effluent liquide C₈⁺ (xylènes, éthylbenzène et C₈⁺) est envoyé dans au moins un réacteur 8 de traitement à la terre activée (attapulgite par exemple). À la sortie de celui-ci, un effluent liquide est introduit par une ligne 9 dans un séparateur 11 d'où sont extraits, en fond, de l'orthoxylène et des hydrocarbures lourds C₈⁺ par une ligne 12. L'orthoxylène peut être éventuellement séparé en tête dans un autre séparateur qui n'est pas représenté sur la figure.

En tête du séparateur, une ligne 13 recueille une fraction contenant principalement des composés aromatiques à 8 atomes de carbone par molécule, les xylènes et l'éthylbenzène et l'introduit dans une zone d'enrichissement 14 en para-xylène, par exemple une zone d'adsorption sélective sur tamis moléculaire opérant en lit mobile simulé, desorbée par du toluène ou du paradiéthylbenzène. Une ligne 15 récupère un extrait contenant le para-xylène qui peut être ultérieurement purifié par cristallisation à au moins un étage, à haute température, de préférence à deux étages entre + 10 et -25°C. Une fraction appauvrie en para-xylène et débarrassée du solvant est envoyée par une ligne 18 dans une zone d'isomérisation 19 opérant éventuellement en présence d'hydrogène, d'où on recueille par une ligne 20 un isomérat. Celui-ci contient des hydrocarbures C₅⁻, des hydrocarbures C₇⁻ et une fraction C₈⁺ et est envoyé par la ligne 20 dans au moins une zone de séparation 21 (deux par exemple qui ne sont pas représentées sur la figure, une enlevant les hydrocarbures C₅⁻ et une autre enlevant les hydrocarbures C₇⁻ (déheptaniseur secondaire) dont le benzène et le toluène par une ligne 22 en tête de la zone 21. En fond, on récupère un effluent contenant les xylènes, l'éthylbenzène et des composés à plus de 8 atomes de carbone, par une ligne 23 qui l'introduit dans au moins un réacteur 24 de traitement à la terre. L'effluent d'isomérisation traité à la terre débarrassé de la majorité des oléfines est recyclé par une ligne 10 connectée à la ligne 9 vers le séparateur 11 en amont de la zone d'adsorption 14.

La fraction enrichie en para-xylène provenant de la zone d'adsorption sélective 14 via la ligne 15 peut être introduite après séparation du solvant dans une zone de purification qui est une zone de cristallisation 16 à un ou deux étages à haute température (+ 10 à - 25°C par exemple). La suspension de cristaux délivrée est séparée par au moins une centrifugeuse (non représentée sur la figure) et des cristaux purifiés à 99,9 % sont recueillis par une ligne 17. Ces étages de purification du para-xylène sont décrits dans les demandes de brevets de la Demanderesse citées ci-avant. Une liqueur mère résultant de l'étape de centrifugation est recueillie, celle de l'étage le plus froid de cristallisation quand il y en a deux, et peut être recyclée soit à l'entrée de la zone d'adsorption 14 via des lignes 25, 25 a, soit à l'entrée de la zone de séparation 11 via des lignes 25, 25 b, soit à l'entrée du réacteur de terre activée via des lignes 25, 25 c, soit à l'entrée du séparateur 21 via des lignes 25, 25d.

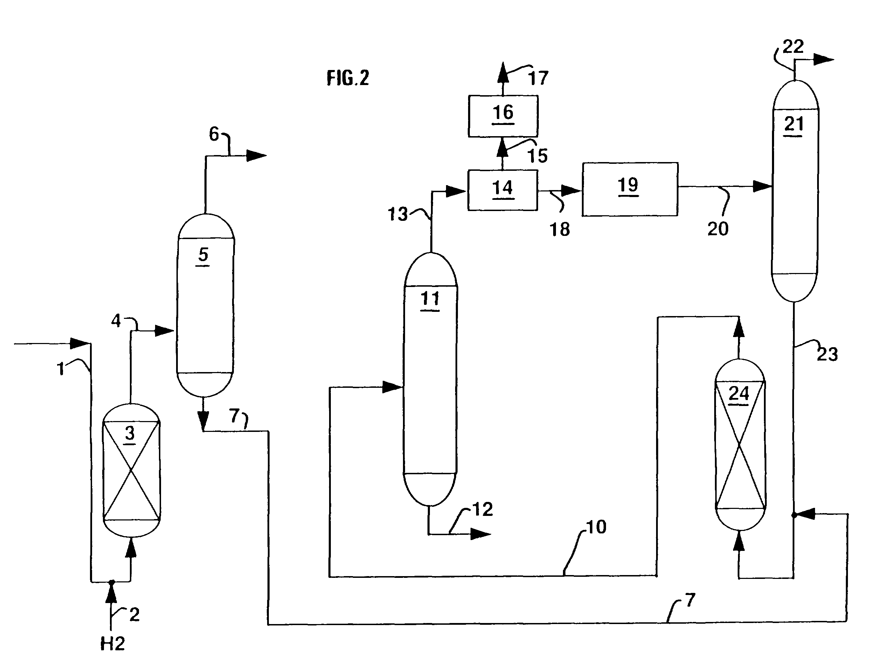

La figure 2 reproduit les mêmes organes avec les mêmes références que celles de la figure 1. Selon cette figure, la charge 1, hydrogénée sélectivement et débarrassée de l'hydrogène en excès c'est-à-dire l'effluent en sortie du séparateur 5 est introduit par la ligne 7 dans le réacteur de traitement à la terre 24. Dans ce réacteur, est introduit aussi par la ligne 23 l'isomérat d'où ont été éliminés les composés légers par la ligne 22. Le mélange ainsi traité à la terre est envoyé par la ligne 10 dans le séparateur 11.

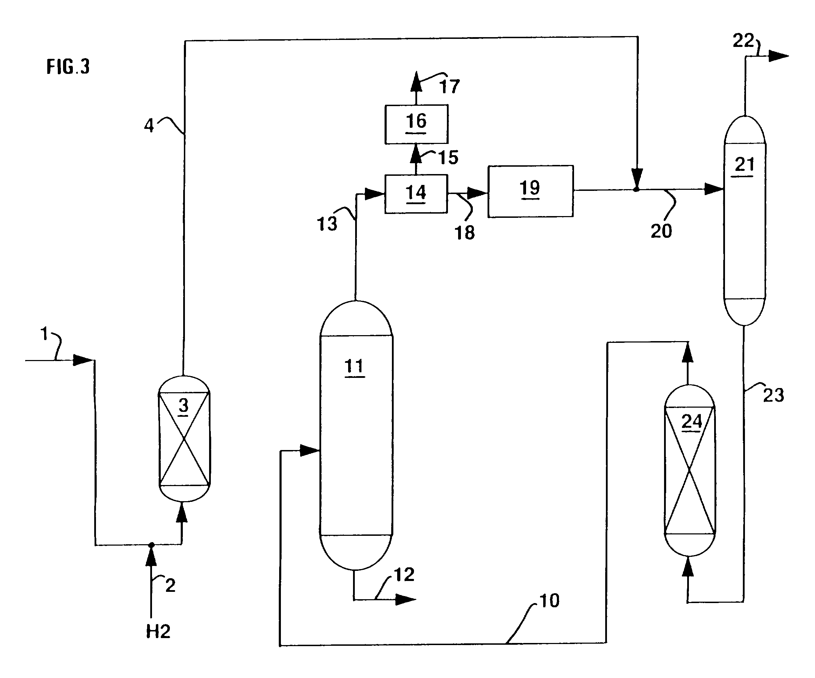

Selon la figure 3, qui reproduit les mêmes organes avec les mêmes références que celles de la figure 1, la charge après avoir été hydrogénée sélectivement est débarrassée de l'hydrogène en excès dans le séparateur qui traite aussi l'isomérat brut. En d'autres termes, l'effluent d'hydrogénation est introduit par la ligne 4 dans la ligne 20 qui conduit l'isomérat dans le séparateur 21. La ligne 23 recueillant l'effluent lourd du séparateur 21 alimente le réacteur 24 de traitement à la terre. L'effluent du réacteur à la terre est introduit comme précédemment par la ligne 10 dans le séparateur 11, qui soutire les composés lourds indésirables.

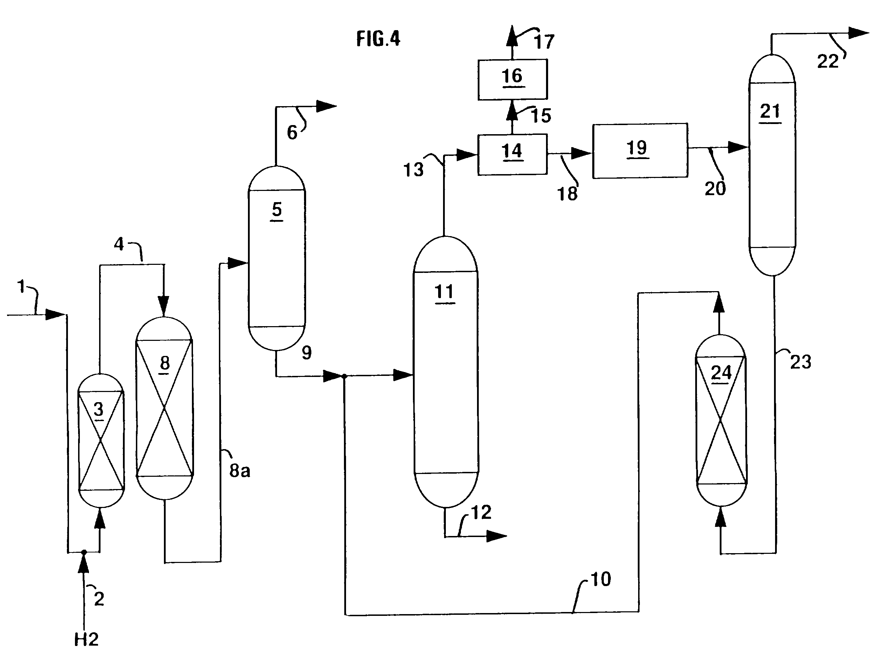

La figure 4 est sensiblement la même que la figure 1 sauf que l'effluent d'hydrogénation via la ligne 4 est introduit dans le réacteur 8 de traitement à la terre avant d'être introduit par une ligne 8a dans le séparateur 5 pour soutirer les composés légers et l'hydrogène.

L'effluent hydrogéné traité à la terre et débarrassé des composés légers est ensuite introduit par la ligne 9 qui reçoit aussi par la ligne 10 l'isomérat traité à la terre dans le séparateur 11. L'effluent léger du séparateur 11 est traité de la même manière que celui de la figure 1.

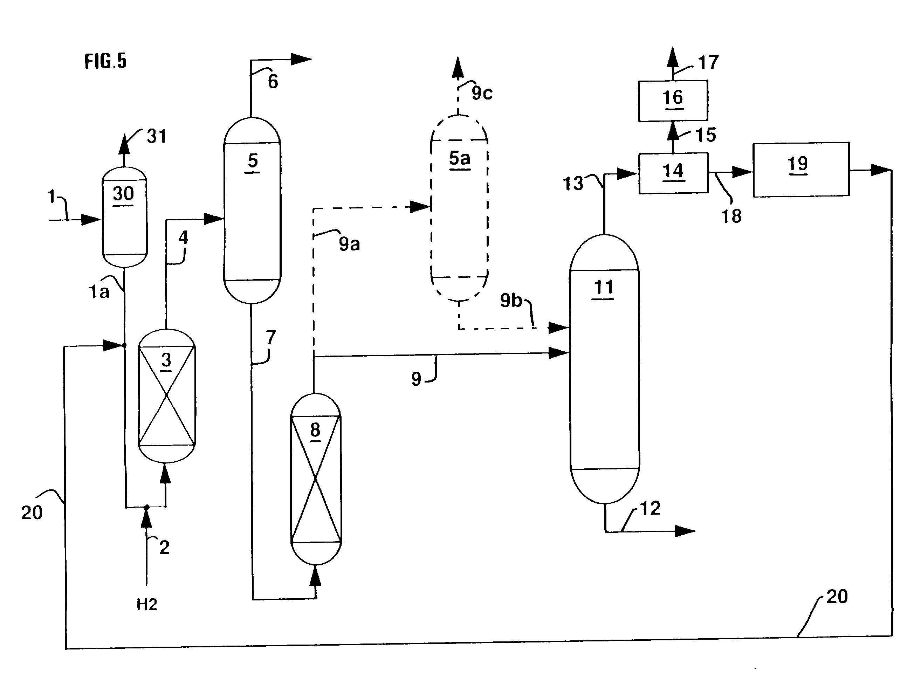

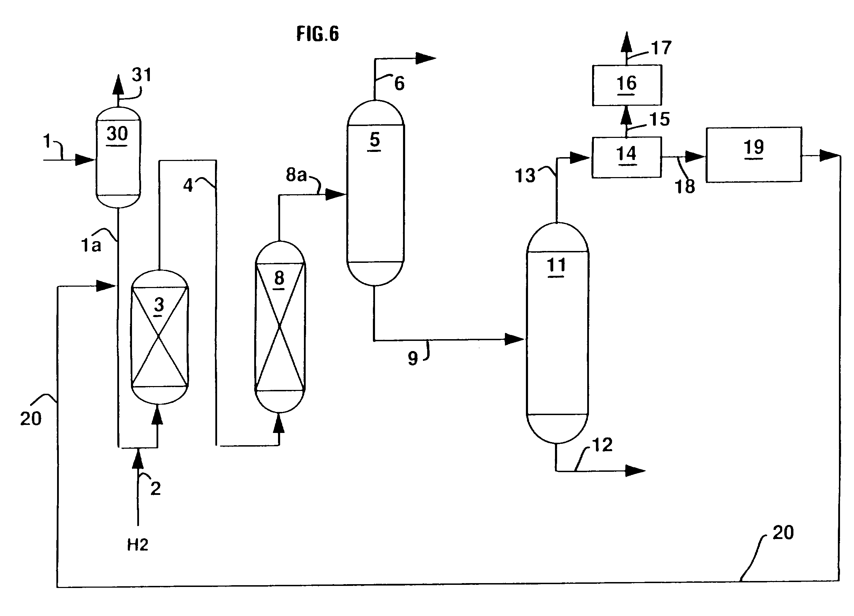

Les figures 5 et 6 illustrent les cas où à la fois la charge et l'isomérat sont hydrogénés sélectivement, débarrassés de l'hydrogène en excès avant ou après un traitement à la terre. Ainsi, sur la figure 5, la charge introduite par la ligne 1 est débarrassée des composés C₃⁻, C₄⁻ et C₅⁻ dans au moins un séparateur 30. L'effluent recueilli par la ligne la est mélangé à l'isomérat brut amené par la ligne 20 et le tout est introduit dans le réacteur d'hydrogénation 3 sélective qui reçoit de l'hydrogène par la ligne 2. L'effluent d'hydrogénation recueilli par la ligne 4 est débarrassé des composés légers C₇⁻ et de l'hydrogène en excès dans le séparateur 5 tandis que les composés les plus lourds sont envoyés par la ligne 7 dans les réacteurs 8 de traitement à la terre activée. L'effluent des réacteurs 8 débarrassé des dioléfines et de la majorité des monooléfines est envoyé au moins en partie par la ligne 9 dans le séparateur 11 pour éliminer les composés les plus lourds tandis que les composés aromatiques C8 récupérés en tête, sont introduits dans la zone d'adsorption 14 de laquelle est recueillie la fraction enrichie en para-xylène qui peut être purifiée par cristallisation 16. La fraction appauvrie en para-xylène est recueillie par la ligne 18 qui l'envoie dans la zone d'isomérisation 19. L'isomérat brut est récupéré par la ligne 20 qui le recycle vers le réacteur 3 d'hydrogénation sélective comme indiqué ci-avant.

De façon préférée, les composés C₃⁻ peuvent être séparés dans le séparateur 30 et les composés C₄⁻ et C₅⁻ ainsi que l'hydrogène en excès dans le séparateur 5. Dans ce cas, l'effluent du réacteur à la terre est envoyé par une ligne 9a dans un autre séparateur (en pointillé) Sa qui délivre en tête par une ligne 9c du benzène et du toluène et en fond par une ligne 9b, les composés C₈⁺ qui alimentent le séparateur 11. L'hydrogène étant séparé en amont du réacteur à la terre 8, on peut ainsi obtenir une meilleure récupération des composés C₈⁺ en aval de ce réacteur 8.

Selon la figure 6, les mêmes organes réalisant les mêmes fonctions sont représentés avec les mêmes références que celles des figures 1 à 5. On dispose du même agencement que celui de la figure 5 sauf que le séparateur 5 d'hydrogène et de composés légers C₇⁻ est situé entre le réacteur de traitement à la terre 8 et le séparateur 11. Ainsi l'effluent hydrogéné et traité à la terre est récupéré du réacteur 8 par une ligne 8a qui le conduit dans le séparateur 5 d'où l'hydrogène notamment est éliminé et l'effluent plus lourd C₈⁺ est introduit par la ligne 9 dans le séparateur 11 duquel sont soutirés les composés lourds C₉⁺ et éventuellement l'orthoxylène.

## Revendications

1. Procédé de séparation et de récupération du p-xylène à partir d'une charge à traiter contenant un mélange de xylènes, dans lequel on fait circuler une partie au moins d'une charge contenant un mélange de xylènes dans une zone dite d'enrichissement (14) adaptée à enrichir une première fraction en p-xylène et délivrant une deuxième fraction appauvrie en p-xylène, on fait circuler ladite deuxième fraction dans une zone d'isomérisation (19) et on récupère un isomérat (20) que l'on recycle vers la zone d'enrichissement, le procédé étant caractérisé en ce que l'on fait circuler au moins une partie choisie dans le groupe formé par la charge initiale, l'isomérat et leur mélange, dans au moins une zone d'hydrogénation sélective (3) en présence d'hydrogène puis dans au moins une zone (8) de traitement à la terre, on réalise une étape de séparation (5) de l'hydrogène en excès dans au moins une zone de séparation, avant ou après ledit traitement à la terre, et on récupère un effluent que l'on envoie dans la zone d'enrichissement (14).

2. Procédé selon la revendication 1, dans lequel l'isomérat et la charge à traiter sont hydrogénées dans la zone (3) d'hydrogénation, puis soumis au traitement à la terre (8), avant ou après séparation (5) de l'hydrogène puis l'effluent résultant est envoyé dans la zone d'enrichissement (14).

3. Procédé selon la revendication 1, dans lequel la charge à traiter est hydrogénée dans la zone (3) d'hydrogénation, puis soumis à un traitement (8) à la terre, avant ou après séparation (5) de l'hydrogène, et dans lequel l'isomérat (20) est recyclé entre les zones d'hydrogénation et de traitement à la terre, et on récupère l'effluent qui est envoyé dans la zone d'enrichissement (14).

4. Procédé selon la revendication 1, dans lequel la charge à traiter est hydrogénée dans la zone (3) d'hydrogénation, puis soumis a un traitement à la terre avant ou après séparation de l'hydrogène et dans lequel l'isomérat (20) est recyclé après la zone de traitement (8) à la terre de la charge hydrogénée, ledit isomérat ayant préalablement subi un traitement à la terre dans une zone de traitement (24) différente.

5. Procédé selon la revendication 1, dans lequel l'isomérat (20) est hydrogéné dans la zone (3) d'hydrogénation, puis soumis au traitement (8) à la terre, avant ou après séparation de l'hydrogène, et dans lequel la charge à traiter, d'où on a éliminé au moins en partie des dioléfines, est introduite en avant de la zone d'enrichissement (14).

6. Procédé selon l'une des revendications 1 à 5, caractérisé en ce que dans la dite zone de séparation, on effectue la séparation de l'hydrogène simultanément à une séparation des composés C₇⁻.

7. Procédé selon l'une des revendications 1 à 5, dans lequel on effectue la séparation de l'hydrogène dans une première zone de séparation, puis la séparation des composés C₇⁻ dans au moins une autre zone de séparation.

8. Procédé selon l'une des revendications 1 à 5, dans lequel on effectue la séparation de l'hydrogène dans une première zone de séparation, puis la séparation des composés C₅⁻ dans une deuxième zone de séparation, et la séparation des composés C₇⁻ restants dans une troisième zone de séparation.

9. Procédé selon l'une des revendications 1 à 5, dans lequel on effectue la séparation de l'hydrogène dans un première zone de séparation, puis la séparation des composés C₄⁻ dans une deuxième zone de séparation, et la séparation des composés C₇⁻ restants dans une troisième zone de séparation.

10. Procédé selon l'une des revendications précédentes, dans lequel on effectue une séparation des composés C₇⁻ après l'étape d'isomérisation.

11. Procédé selon l'une des revendications précédentes, dans lequel la charge à traiter est choisie dans le groupe formé par un réformat brut, un réformat brut d'où ont été éliminés au moins en partie les composés C₃⁻, un réformat brut d'où ont été éliminés au moins en partie les composés C₄⁻, un réformat brut d'où ont été éliminés les composés C₅⁻, une coupe C₈ aromatique contenant une majeure partie de xylènes, et leurs mélanges.

12. Procédé selon l'une des revendications précédentes, dans lequel on fait circuler l'effluent à enrichir dans au moins un séparateur (11) situé à l'entrée de la zone d'enrichissement.

13. Procédé selon l'une des revendications précédentes dans lequel la zone d'enrichissement en para-xylène est au moins une zone de cristallisation à très basse température qui délivre, après une étape de séparation, un liquide (la deuxième fraction) que l'on isomérise dans la zone d'isomérisation et des cristaux que l'on fond qui sont une partie au moins de ladite première fraction.

14. Procédé selon l'une des revendications 1 à 12 dans lequel la zone d'enrichissement en para-xylène est une zone d'adsorption sélective contenant un adsorbant zéolithique, on réalise une adsorption sélective de la charge, en présence d'un solvant de désorption, on récupère ladite première fraction enrichie en para-xylène et la seconde fraction appauvrie en para-xylène, on isomérise ladite seconde fraction dans la zone d'isomérisation contenant un catalyseur d'isomérisation dans des conditions adéquates pour produire un isomérat contenant du para-xylène et on recycle au moins en partie l'isomérat vers la zone d'adsorption.

15. Procédé selon l'une des revendication précédentes, dans lequel l'hydrogénation sélective est effectuée en présence d'un catalyseur contenant au moins un élément du groupe VIII dans lequel du soufre a été introduit préalablement au passage de la charge à hydrogéner.

16. Procédé selon la revendication 15, dans lequel le soufre a été introduit par traitement du catalyseur au moyen d'un agent soufré dans la zone d'hydrogénation.

17. Procédé selon la revendication 15, dans lequel le soufre a été introduit dans le catalyseur au moyen d'un agent soufré, préalablement au chargement dudit catalyseur dans la zone d'hydrogénation.

18. Procédé selon la revendication 17, dans lequel au moins un agent soufré à base d'un composé organique renfermant du soufre est incorporé dans le catalyseur préalablement à son emploi et préalablement à son activation par de l'hydrogène.

19. Procédé selon l'une des revendications 15, 17 ou 18, dans lequel le catalyseur contient de 0,1 à 1 % (poids par rapport au support) de palladium, ledit catalyseur ayant été traité avant son activation par ledit agent soufré de façon à introduire de 0,05 à 1 % (poids par rapport à la masse) de soufre.

## Patentansprüche

1. Verfahren zur Trennung und Gewinnung des p-Xylols ausgehend von einer zu behandelnden Charge, welche eine Mischung von Xylolen enthält, in der man einen Teil mindestens einer Charge zirkulieren lässt, welche eine Mischung von Xylolen in einer sogenannten Zone der Anreicherung (14) enthält, so ausgelegt, dass eine erste Fraktion an p-Xylol angereichert und das eine zweite, an p-Xylol verarmte Fraktion abgegeben wird, wobei man die zweite Fraktion in einer Isomerisierungszone (19) zirkulieren lässt und man ein Isomerat (20) sammelt, das man gegen die Anreicherungszone rezycliert, **dadurch gekennzeichnet,** dass man mindestens einen gewählten Anteil aus der von der Anfangscharge, dem Isomerat und seiner Mischung gebildeten Gruppe zirkulieren lässt, in mindestens einer Zone selektiver Hydrierung (3) in Gegenwart von Wasserstoff, man dann in mindestens einer Bleicherdebehandlungszone (8) einen Trennungsschritt (5) des überschüssigen Wasserstoffs in mindestens einer Trennzone, vor oder nach dieser Bleicherdebehandlung, umsetzt und man einen Abstrom aufnimmt, den man in die Anreicherungszone (14) führt.

2. Verfahren nach Anspruch 1, in dem das Isomerat und die zu behandelnde Charge in der Hydrierungszone (3) hydriert werden, dann der Bleicherdebehandlung (8), vor oder nach der Trennung des Wasserstoffs (5) unterzogen werden, dann der resultierende Abstrom in die Anreicherungszone (14) geführt wird.

3. Verfahren nach Anspruch 1, in dem die zu behandelnde Charge in der Hydrierungszone (3) hydriert wird, dann einer Bleicherdebehandlung (8) unterzogen wird, vor oder nach der Trennung des Wasserstoffs (5), und in dem das Isomerat (20) zwischen den Zonen der Hydrierung und der Bleicherdebehandlung rezycliert wird, und man den Abstrom sammelt, der in die Anreicherungszone (14) geführt wird.

4. Verfahren nach Anspruch 1, in dem die zu behandelnde Charge in der Hydrierungszone (3) hydriert wird, dann einer Bleicherdebehandlung (8) unterzogen wird, vor oder nach der Trennung des Wasserstoffs (5), und in dem das Isomerat (20) nach der Bleicherdebehandlungszone (8) der hydrierten Charge rezycliert wird, wobei das Isomerat vorher einer Bleicherdebehandlung in einer anderen Behandlungszone (24) unterzogen wurde.

5. Verfahren nach Anspruch 1, in dem das Isomerat (20) in der Hydrierungszone (3) hydriert wird, dann der Bleicherdebehandlung (8) unterzogen wird, vor oder nach der Trennung des Wasserstoffs, und in dem die zu behandelnde Charge, wovon man mindestens einen Anteil der Diolefine entfernt hat, vor der Anreicherungszone (14) eingeführt wird.

6. Verfahren nach einem der Ansprüche 1 bis 5, **dadurch gekennzeichnet,** dass man in der Trennzone die Trennung des Wasserstoffs gleichzeitig mit einer Trennung von C₇-Verbindungen ausführt.

7. Verfahren nach einem der Ansprüche 1 bis 5, in dem man die Trennung des Wasserstoffs in einer ersten Trennzone und dann die Trennung der C₇-Verbindungen in einer anderen Trennzone ausführt.

8. Verfahren nach einem der Ansprüche 1 bis 5, in dem man die Trennung des Wasserstoffs in einer ersten Trennzone, dann die Trennung von C₅-Verbindungen in einer zweiten Trennzone und dann die Trennung der verbleibenden C₇-Verbindungen in einer dritten Trennzone ausführt.

9. Verfahren nach einem der Ansprüche 1 bis 5, in dem man die Trennung des Wasserstoffs in einer ersten Trennzone, dann die Trennung von C₄-Verbindungen in einer zweiten Trennzone und dann die Trennung der verbleibenden C₇-Verbindungen in einer dritten Trennzone ausführt.

10. Verfahren nach einem der vorhergehenden Ansprüche, in dem man Trennung der C₇-Verbindungen nach dem Isomerisierungsschritt ausführt.

11. Verfahren nach einem der vorhergehenden Ansprüche, in dem die zu behandelnde Charge aus einer, aus einem Bruttoreformat, einem Bruttoreformat, wovon zumindest teilweise C₃-Verbindungen entfernt wurden, einem Bruttoreformat, wovon zumindest teilweise C₄-Verbindungen entfernt wurden, einem Bruttoreformat, wovon zumindest teilweise C₅-Verbindungen entfernt wurden, einem Schnitt von C₈-Aromaten, welcher einen Hauptteil von Xylolen enthält, und deren Mischungen gebildeten Gruppe gewählt wird.

12. Verfahren nach einem der vorhergehenden Ansprüche, in dem man den anzureichernden Abstrom in mindestens einem, am Eingang der Anreicherungszone gelegenen Abscheider (11) zirkulieren läßt.

13. Verfahren nach einem der vorhergehenden Ansprüche, in dem die Anreicherungszone an p-Xylol mindestens eine Kristallisationszone von sehr niedriger Temperatur ist, die nach einem Trennschritt eine Flüssigkeit(die zweite Fraktion), welche man in der Isomerisierungszone isomerisiert und Kristalle, welche man löst und die ein Teil, zumindest der ersten Fraktion sind, liefert.

14. Verfahren nach einem der Ansprüche 1 bis 12, in dem die Anreicherungszone an p-Xylol eine selektive Adsorptionszone ist, welche ein zeolithisches Adsorbens enthält, wobei man die eine selektive Adsorption der Charge in Gegenwart eines Desorptionslösungsmittels umsetzt, man die erste, an p-Xylol angereicherte Fraktion und die zweite an p-Xylol verarmte Fraktion sammelt, man die zweite Fraktion in der Isomerisierungszone, welche einen Isomerisierungskatalysator enthält, unter passenden Bedingungen zum Erzeugen eines para-Xylol enthaltenden Isomerates isomerisiert und man zumindest teilweise das Isomerat zur Adsorptionszone rezycliert.

15. Verfahren nach einem der vorhergehenden Ansprüche, in dem man die selektive Hydrogeniereung in Gegenwart eines, mindestens ein Element der Gruppe VIII enthaltenden, Katalysators ausführt, in den Schwefel vor dem Durchgang der zu hydrgenierenden Charge eingeführt worden ist.

16. Verfahren nach Anspruch 15, in dem der Schwefel durch Behandlung des Katalysators mittels eines schwefelhaltigen Reagenzes in die Hydrierungszone eingeführt worden ist.

17. Verfahren nach Anspruch 15, in dem der Schwefel in den Katalysator mittels eines schwefelhaltigen Reagenzes vor Beladung des Katalysators in die Hydrierungszone eingeführt worden ist.

18. Verfahren nach Anspruch 17, in dem zumindest ein schwefelhaltiges Reagenz auf Basis einer schwefelhaltigen organischen Verbindung in den Katalysator vor seinem Gebrauch und vor seiner Aktivierung durch den Wasserstoff eingebaut wird.

19. Verfahren nach einem der Ansprüche 15, 17 oder 18, in dem der Katalysator 0.1 bis 1% (Gewicht bezüglich des Trägers) Palladium enthält, wobei der Katalysator vor seiner Aktivierung durch das schwefelhaltige Reagenz in einer Weise behandelt worden ist, um 0.05 bis 1 % (Gewicht bezüglich der Masse) Schwefel einzuführen.

## Claims

1. A process for the separation and recovery of p-xylene from a feed to be treated containing a mixture of xylenes, in which at least a portion of a feed containing a mixture of xylenes is circulated in a zone termed the enrichment zone (14) to enrich a first fraction (15) in p-xylene and to provide a second fraction (18) which is depleted in p-xylene, said second fraction being circulated in an isomerisation zone (19) and an isomerate (20) being recovered which is recycled to the enrichment zone, the process being characterized in that at least a portion selected from the group formed by the initial feed, the isomerate and a mixture thereof, is circulated in at least one selective hydrogenation zone (3) in the presence of hydrogen then in at least one clay treatment zone (8), a step (5) for the separation of excess hydrogen being carried out in at least one separation zone before or after said clay treatment, and an effluent is recovered which is sent to the enrichment zone (14).

2. A process according to claim 1, in which the isomerate and the feed to be treated are hydrogenated in hydrogenation zone (3) then treated with clay (8) before or after separation of hydrogen (5) then the resulting effluent is sent to the enrichment zone (14).

3. A process according to claim 1, in which the feed to be treated is hydrogenated in hydrogenation zone (3) then treated with clay (8) before or after separation of hydrogen (5), the isomerate (20) being recycled between the hydrogenation and clay treatment zones, and the effluent is recovered and sent to the enrichment zone (14).

4. A process according to claim 1, in which the feed to be treated is hydrogenated in hydrogenation zone (3) then treated with clay before or after separation of hydrogen and in which the isomerate (20) is recycled downstream of the clay treatment zone (8) for the hydrogenated feed, the isomerate having been clay treated in a different treatment zone (24).

5. A process according to claim 1, in which the isomerate (20) is hydrogenated in the hydrogenation zone (3) then clay treated (8) before or after hydrogen separation, and in which the feed to be treated, from which at least a portion of the diolefins has been eliminated, is introduced upstream of the enrichment zone (14).

6. A process according to any one of claims 1 to 5, characterized in that in said separation zone, hydrogen separation is carried out simultaneously with separation of C7-compounds.

7. A process according to any one of claims 1 to 5, in which hydrogen separation is carried out in a first separation zone, then the C7- compounds are separated in at least one further separation zone.

8. A process according to any one of claims 1 to 5, in which hydrogen separation is carried out in a first separation zone, then the C5- compounds are separated in a second separation zone, and the remaining C7- compounds are separated in a third separation zone.

9. A process according to any one of claims 1 to 5, in which H2 separation is carried out in a first separation zone, then the C4- compounds are separated in a second separation zone, and the remaining C7- compounds are separated in a third separation zone.

10. A process according to any one of the preceding claims, in which separation of the C7-compounds is carried out after the isomerisation step.

11. A process according to any one of the preceding claims, in which the feed to be treated is selected from the group formed by a crude reformate, a crude reformate from which at least a portion of the C3- compounds has been eliminated, a crude reformate from which at least a portion of the C4- compounds has been eliminated, a crude reformate from which at least a portion of the C5- compounds have been eliminated, or a C8 cut rich in aromatic compounds containing a major portion of xylenes, or mixtures thereof

12. A process according to any one of the preceding claims, in which the effluent to be enriched is circulated in at least one separator (11) located at the inlet to the enrichment zone.

13. A process according to any one of the preceding claims, in which the para-xylene enrichment zone is formed by at least one very low temperature crystallization zone which, after a separation step, produces a liquid (the second fraction) which is isomerised in the isomerisation zone, and crystals which are melted which form at least a portion of the first fraction.

14. A process according to any one of claims 1 to 12, in which the para-xylene enrichment zone is formed by a selective adsorption zone containing a zeolitic adsorbent, the feed is selectively adsorbed in the presence of a desorption solvent, the first fraction, which is enriched in para-xylene, and the second fraction, which is depleted in para-xylene, are recovered, the second fraction is isomerised in the isomerisation zone which contains an isomerisation catalyst under conditions suitable for the production of an isomerate containing para-xylene, and at least a portion of the isomerate is recycled to the adsorption zone.

15. A process according to any one of the preceding claims, in which selective hydrogenation is carried out in the presence of a catalyst containing at least one element from group VIII into which sulphur has been introduced prior to the passage of the feed to be hydrogenated.

16. A process according to claim 15, in which sulphur is introduced by treating the catalyst with a sulphur-containing agent in the hydrogenation zone

17. A process according to claim 15, in which sulphur is introduced into the catalyst by means of a sulphur-containing agent introduced prior to charging said catalyst into the hydrogenation zone.

18. A process according to claim 17, in which at least one sulphur-containing agent based on an organic compound containing sulphur can be incorporated into the catalyst prior to its use and prior to its activation by hydrogen.

19. A process according to any one of claims 15, 17 or 18, in which the catalyst contains 0.1% to 1% (by weight with respect to the support) of palladium, said catalyst having been treated with said sulphur-containing agent before activation to introduce 0.05% to 1% (by weight with respect to the mass) of sulphur.
